# EUROPEAN PATENT APPLICATION

(11) **EP 2 687 527 A1**
(43) Date of publication of application: **22.01.2014**
(21) Application number: 12382291.8
(22) Date of filing: 19.07.2012
(51) Int. Cl.: C07D 453/04, B01J 31/02

(54) **Cinchona alkaloid derivatives, their process of preparation and their use as catalysts**

(71) Applicant: Fundació Privada Institut Català d'Investigació Química (ICIQ), 43007 Tarragona (ES); Institució Catalana de Recerca i Estudis Avançats, 08010 Barcelona (ES)
(72) Inventor: Melchiorre, Paolo, 43007 Tarragona (ES); Bravo Lara, Fernando, 43800 Valls (ES); Martin, Rafael, 43005 Tarragona (ES)
(74) Representative: ZBM Patents

(57) **Abstract**

The present invention provides a process for preparing a substantially stereochemically pure compound of formula (I), or a salt thereof wherein R₁ and R₂ are independently selected from the group consisting of H, -CH₂-CH₃, and -CH=CH₂, the process comprising the step of demethylating the corresponding methylated compound of formula (II) in the presence of an alkaline or alkaline earth metal (C₁-C₁₀) alkylthiolate salt and a polar aprotic solvent.

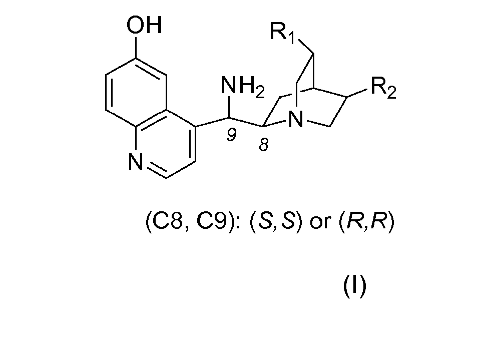

## Description

The present invention refers to a process for preparing cinchona alkaloid derivatives. Furthermore, the present invention provides a new cinchona derivative of formula (Id) which is useful as catalyst.

### BACKGROUND ART

Asymmetric organocatalysis has emerged as a new and powerful methodology for the catalytic production of enantiomerically pure organic compounds and also as one of the most rapidly growing and competitive research areas in synthetic organic chemistry, in particular with respect to activation of carbonyl groups via mechanisms involving imine or enamine formation. Organocatalysis has attracted considerable attention in chemistry in recent years because it involves metal-free catalysts. This approach might become valuable for the preparation of pharmaceutical compounds, in which metal contamination of the product cannot be tolerated and for which diastereo- and enantioselectivities are important issues.

Small enantiopure organic molecules, such as proline and cinchona alkaloids derivatives, can function as catalytically active species. Indeed, in the last two decades, cinchona alkaloids have become widely used as catalysts in asymmetric synthesis to give access to chiral building blocks of high enantiopurity. Particularly, the primary amines 9-amino(9-deoxy)epi cinchona alkaloids **A-G** (Scheme 1), prepared from naturally occurring quinine, dihydroquinine, quinidine or dihydroquinidine, have been recognized as a reliable and general catalyst class with a high synthetic potential for aminocatalysis. The cinchona-based primary amines, which combine a primary amine functionality and a Lewis base in the same structure, have enabled the stereoselective functionalisation of a variety of sterically hindered carbonyl compounds, which cannot be functionalised using secondary amines and which are challenging substrates for metal catalysis as well. Remarkably, this single catalyst class can activate very structurally different carbonyl compounds (*e.g*. simple ketones as well as α-branched substituted aldehydes and ketones or their α,β-unsaturated counterparts) with consistently high levels of stereocontrol, while making use of the different aminocatalytic activation modes: iminium ion, enamine, dienamine and trienamine activations.

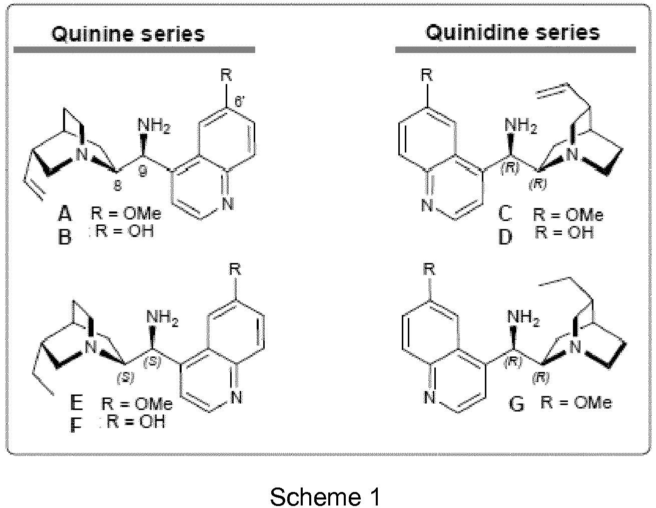

It is remarkable that hydrochloride salts of the cinchona alkaloids **A**, **C**, and **E** are currently available from commercial suppliers, although at somehow prohibitive prices.

Due to the increasing interest for such cinchona derivatives, several synthetic strategies for their preparation have been disclosed. The most common strategy, starting from the natural products quinine, dihydroquinine, dihydroquinidine or quinidine, is based on performing a Mitsunobu reaction that leads to the corresponding C9-azido compound by a S_{N}2 mechanism. The reduction is performed *in situ* by adding triphenylphosphine (Staudinger reaction). Hydrolysis of the intermediate aminophosphorane yields the free amine:

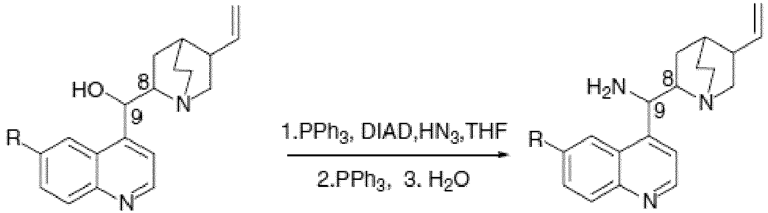

When the cinchona alkaloid to be obtained is either **B**, **D** or **F**, from **A**, **C** or **E**, respectively, a demethylation step is required. In this regard, the used demethylating agent is BBr₃, which requires working at -78 °C.

Some drawbacks associated to the Mitsunobu reaction are: (a) the requirement of a "HN₃" source, which is either extremely toxic (hydrazoic acid itself) or very expensive (diphenylphosphoryl azide (DPPA)); (b) the use of diisopropylazodicarboxylate (DIAD), which is very expensive; and (c) DPPA, DIAD and PPh₃ are molecules of high molecular weight, which do not contribute significantly to the final structrure of the products, this giving rise to a great amount of undesired by-products, leading to a more difficult isolation of the reaction product and high load of waste generation.

In addition, the use of BBr₃ in order to perform the demethylation step at a temperature of -78°C shows further drawbacks. On one hand, said conditions are not feasible in most pilot plant reactors due to the extreme low temperature required and, on the other hand, it is well-known that BBr₃ is extremely corrosive, with the associated handling limitations. A further drawback is that when performing the demethylation with BBr₃,the reaction has some reproducibility issues.

In view of the above, there is still the need of providing alternative processes for the synthesis of cinchona alkaloid derivatives.

### SUMMARY OF THE INVENTION

The present inventors have developed a process for the synthesis of substantially stereochemically pure compounds of formula (I) 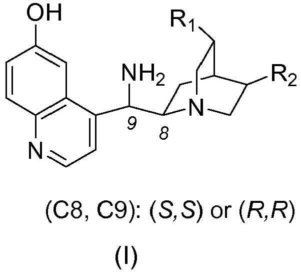 which is based on a demethylation step of the corresponding methylated compound of formula (II):

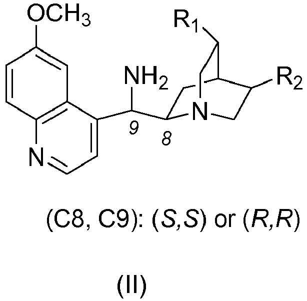

In particular, the present inventors have found that when the demethylation step of the compound of formula (II) is performed in the presence of a thiolate salt and an aprotic polar solvent under the specific conditions of reaction of the invention, the process is easy to implement at industrial scale and cheaper. In addition, the product is stable in the reaction mixture, and the work-up is simpler.

Furthermore, the fact that the demethylation step occurs with a thiolate salt, allows operating the process under mild conditions, which gives a clean reaction profile, free of inorganic by-products. In addition, using a thiolate, the process of the invention also allows avoiding the use of Lewis acids, which are usually sensitive and corrosive reagents.

In view of the above, the process of the invention is highly reproducible in an effective and safe manner.

Thus, in a first aspect the present invention provides a process for preparing a substantially stereochemically pure compound of formula (I), or a salt thereof, 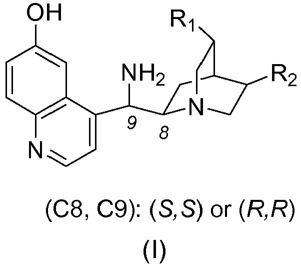 wherein R₁ and R₂ are independently selected from the group consisting of H, -CH₂-CH₃, and -CH=CH₂, in such a way that compound of formula (I) is selected from the group consisting of: 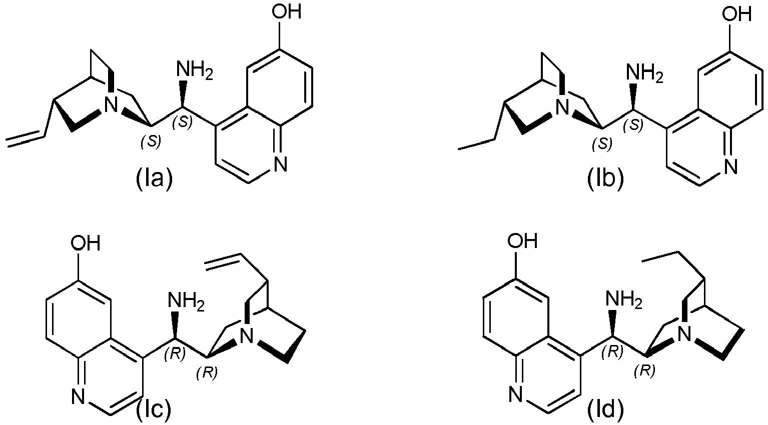 the process comprising the step of demethylating a compound of formula (II), wherein R₁ and R₂ have the same meaning as for compound of formula (I), in the presence of an alkaline or alkaline earth metal (C₁-C₁₀)alkylthiolate salt and a polar aprotic solvent having a polarity index equal to or higher than 6.0, being the molar ratio of thiolate salt:compound of formula (II) comprised of from 2 to 20 equivalents , 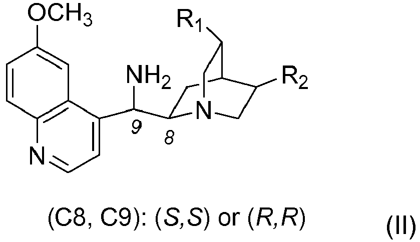 with the proviso that,
when the compound of formula (I) is the compound (Ia), then the compound of formula (II) is the compound (IIa) 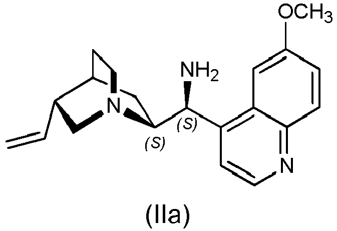 when the compound of formula (I) is the compound (Ib), then the compound of formula (II) is the compound (IIb); 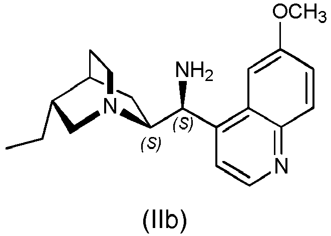 when the compound of formula (I) is the compound (Ic), then the compound of formula (II) is the compound (IIc); and 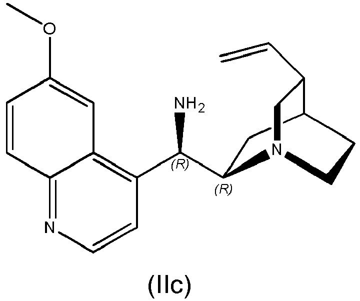 when the compound of formula (I) is the compound (Id), then the compound of formula (II) is the compound (IId).

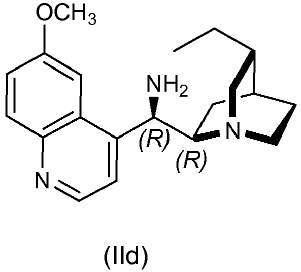

As it has been stated in "background art" section, the demethylation reactions disclosed in the prior art for preparing cinchona alkaloid derivatives comprise the use of BBr₃.

With the process of the invention, wherein the demethylation step takes place with a thiolate salt instead of with BBr₃, the handling problems (due to the corrosive nature of the reagent) as well as the unfeasibility of performing said step at industrial scale (due to the obligation of working at extreme low temperatures) are avoided. In addition, when the thiolate salt is used in the demethylation step, no decomposition of the product is observed and the reproducibility issues are overcome. Therefore, with the process of the invention the compound of fomula (I) is obtained in appropriate yields.

The inventors of the present invention have found that the nature of the starting product, as well as the molar ratio of alkylthiolate salt to reaction substrate and the nature of the solvent, critically determine the success of the process of the invention, as it is shown in Example 12.

In a second aspect, the present invention provides a compound of formula (Id):

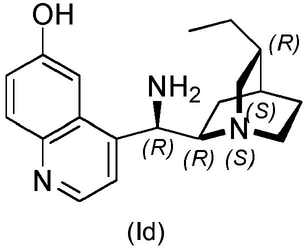

In a third aspect, the present invention provides the use of the compound of formula (Id) as defined in the second aspect of the invention, as a catalyst.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The term "substantially stereochemically pure", when applied to a compound of formula (I), concerns the C9 stereogenic center and has to be understood as a compound (I) having an amount of epimeric by-product at C9 equal to or lower than 5% molar (or weight), the other stereogenic carbon atoms conserving the configuration provided by compound (II).

In the formula of the compounds of the present invention, the use of bold and dashed lines to denote particular configuration of groups follows the IUPAC convention. A bond indicated by a broken line indicates that the group in question is below the general plane of the molecule as drawn (the "alpha" configuration), and a bond indicated by a bold line indicates that the group at the position in question is above the general plane of the molecule as drawn (the "beta" configuration).

As mentioned above, a process for the preparation of cinchona derivatives of formula (I) is provided, which comprises the demethylation of the corresponding methylated compound of formula (II) in the presence of an alkaline or alkaline earth thiolate salt and a polar aprotic solvent.

In one embodiment, the alkaline thiolate salt is selected from lithium thiolate, sodium thiolate, and potassium thiolate. In another embodiment of the process of the invention, the alkaline or alkaline earth thiolate salt is a (C₁-C₁₀)alkanethiolate salt, such as methanethiolate, ethanethiolate or butanethiolate. In still another preferred embodiment, the alkaline thiolate salt is sodium (C₁-C₁₀)alkanethiolate. Preferably, the (C₁-C₁₀)alkanethiolate salt is selected from the group consisting of: sodium ethanethiolate, sodium butanethiolate and sodium t-butanethiolate.

In another embodiment of the process of the invention, the demethylation step is carried out at a temperature comprised from 50 to 110°C. In a further embodiment, the demethylation step is carried out at a temperature comprised of from 80 to 110°C. Particularly, the demethylation step is carried out at a temperature of about 80 °C.

In another embodiment of the process of the invention, the demethylation step is carried out with a molar ratio of thiolate salt to compound of formula (II) comprised of from from 3:1 to 12:1. In a more preferred embodiment, the demethylation step is carried out with a molar ratio of thiolate salt to compound of formula (II) comprised of from 3:1 to 10:1. In a more preferred embodiment, the demethylation step is carried out with a molar ratio of thiolate salt to compound of formula (II) comprised of from 3:1 to 8:1.

In another embodiment of the process of the invention, the demethylation step is carried out in the presence of a polar aprotic solvent having a polarity index equal to or higher than 6.0.

The term "polarity index" is known in the art and has to be understood as a relative measure of the degree of interaction of the solvent with various polar test solutes (Barwick J. V., 1997).

In one embodiment, the polar aprotic solvent used in the demethylation step has a polarity index comprised of from 6.4 to 7.2.

In another embodiment, the polar aprotic solvent used in the demethylation step is selected from dimethylformamide (DMF), dimethylsulfoxide (DMSO), methylpirrolidone (NMP), hexamethylphosphoramide (HMPA), 1,3-Dimethyl-3,4,5,6-tetrahydro-2(1H)-pyrimidinone (DMPU), N-methylimidazole, 1,1,3,3-tetramethylurea (TMU), 1,3-Dimethyl-2-imidazolidinone (DMI), and mixtures thereof. Preferably, the polar aprotic solvent for performing the demethylation step is selected from dimethylformamide, dimethylsulfoxide, and mixtures thereof.

In an embodiment, when performing the demethylation step of the present invention, the polar aprotic solvent is dimethylformamide and the molar ratio of thiolate:compound of formula (II) is comprised from 5:1 to 20:1. In a further embodiment, the polar aprotic solvent is dimethylformamide and the molar ratio of thiolate:compound of formula (II) is comprised from 5:1 to 12:1. In still a further embodiment, the polar aprotic solvent is dimethylformamide and the molar ratio is comprised from 5:1 to 10:1. In still yet another embodiment, the polar aprotic solvent is dimethylformamide and the molar ratio is 5:1.

In another embodiment, when performing the demethylation step of the present invention the polar aprotic solvent is dimethysulfoxide and the molar ratio thiolate:compound of formula (II) is comprised from 3:1 to 20:1. In a further embodiment, the polar aprotic solvent is dimethylsulfoxide and the molar ratio of thiolate:compound of formula (II) is comprised from 3:1 to 12:1. In still a further embodiment, the polar aprotic solvent is dimethylsulfoxide and the molar ratio is comprised from 3:1 to 10:1. In still yet a further embodiment, the polar aprotic solvent is dimethylsulfoxide and the molar ratio is comprised from 3:1 to 8:1.

In a further embodiment, when performing the demethylation step of the present invention, the thiolate salt is sodium ethylthiolate, the polar aprotic solvent is dimethylsulfoxide, the molar ratio of sodium ethylthiolate to the compound of formula (II) is about 6.0, which means a ratio of 6.0 ± 0.4, and the reaction temperature is about 80°C, which means a temperature of 80 ± 3°C.

In a further embodiment, when performing the demethylation step of the present invention, the thiolate salt is sodium ethylthiolate, the polar aprotic solvent is dimethylformamide, the molar ratio of sodium ethylthiolate to the compound of formula (II) is about 6.0, which means a ratio of 6.0 ± 0.4, and the reaction temperature is 110 ± 3°C.

In a further embodiment, when performing the demethylation step of the present invention, the thiolate salt is sodium ethylthiolate, the polar aprotic solvent is dimethylformamide, the molar ratio of sodium ethylthiolate to the compound of formula (II) is about 5.0, which means a ratio of 5.0 ± 0.4, and the reaction temperature is 110 ± 3°C.

In a further embodiment, when performing the demethylation step of the present invention, the thiolate salt is sodium ethylthiolate, the polar aprotic solvent is dimethylsulfoxide, the molar ratio of sodium ethylthiolate to the compound of formula (II) is about 3.0, which means a ratio of 3.0 ± 0.4, and the reaction temperature is 80 ± 3°C.

In still another embodiment, when performing the demethylation step of the present invention, the thiolate salt is sodium t-butylthiolate, the polar aprotic solvent is dimethylsulfoxide, the molar ratio of sodium t-butylthiolate to the compound of formula (II) is about 8, which means a ratio of 8.0 ± 0.4, and the reaction temperature is 80 ± 3°C.

In still another embodiment, when performing the demethylation step of the present invention, the thiolate salt is sodium t-butylthiolate, the polar aprotic solvent is dimethylsulfoxide, the molar ratio of sodium t-butylthiolate to the compound of formula (II) is about 6, which means a ratio of 6.0 ± 0.4, and the reaction temperature is 80 ± 3°C.

In still another embodiment, when performing the demethylation step of the present invention, the thiolate salt is sodium t-butylthiolate, the polar aprotic solvent is dimethylsulfoxide, the molar ratio of sodium t-butylthiolate to the compound of formula (II) is about 5, which means a ratio of 5.0 ± 0.4, and the reaction temperature is 80 ± 3°C.

The following Schemes 2 and 3 illustrate a particular embodiment of the global process starting from commercial compounds. As shown, the global process requires only two inexpensive starting materials for obtaining the cinchona alkaloid derivatives of formula (Ia)-(Id). In addition, the reagents used in the different steps are affordable and less residues are generated. The steps are also performed in reasonably mild conditions, thus allowing for process scale-up. Advantageously, the synthetic route described in Schemes 2 and 3 does not require any purification step of the synthetic intermediates produced that can be used after isolation from the reaction mixtures. The compounds of formula (IIa), (IIb), (IIc) and (IId), being useful as organocatalysts, can be purified and isolated as solid salts. Preferably, the respective salts of compounds of formula (IIa), (IIb), (IIc) and (IId) are isolated by precipitation as described below. Furthermore, the products of formula (IIa), (IIb), (IIc) or (IId) obtained in steps G-H and G'-H' can be used in subsequent steps without isolating them. In the respective transformations of the compounds of formula (IIa), (IIb, (IIc) and (IId) into the compounds of formula (Ia), (Ib), (Ic) and (Id) described in steps E to J for (Ia) and (Ib) and E' to J' for (Ic) and (Id), the steps E to H and E' to H' can be omitted.

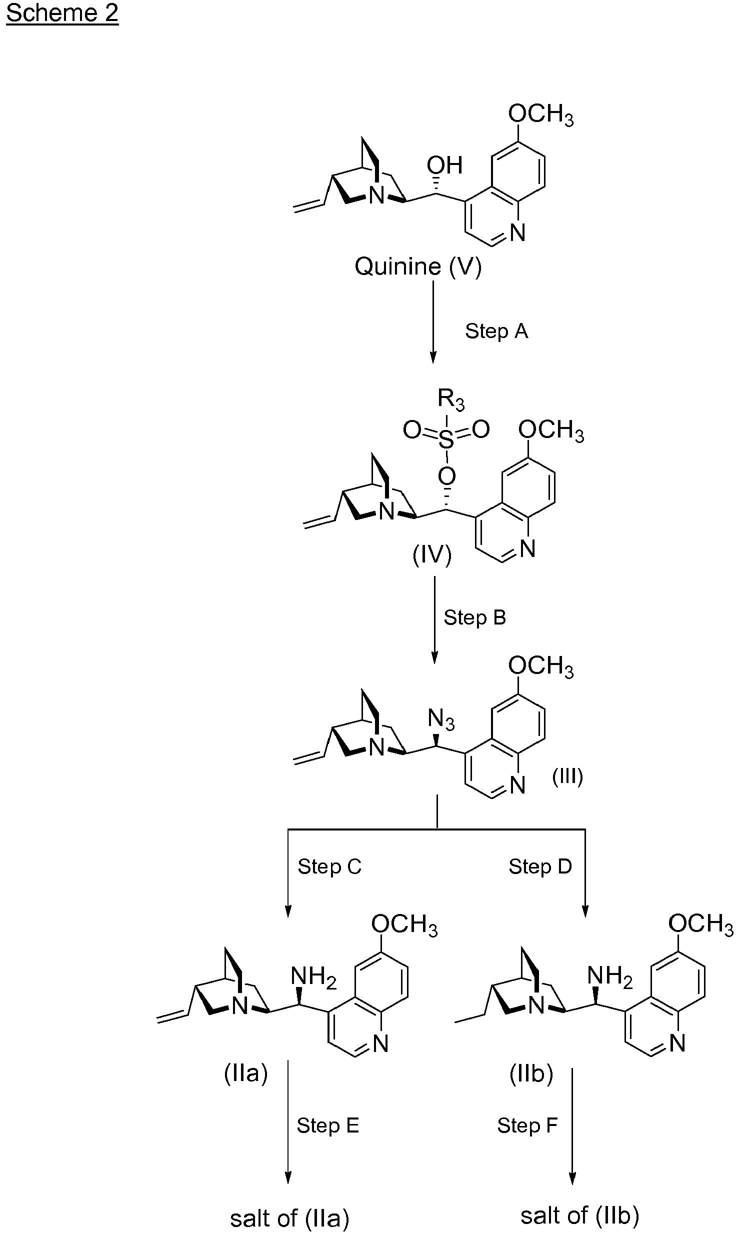

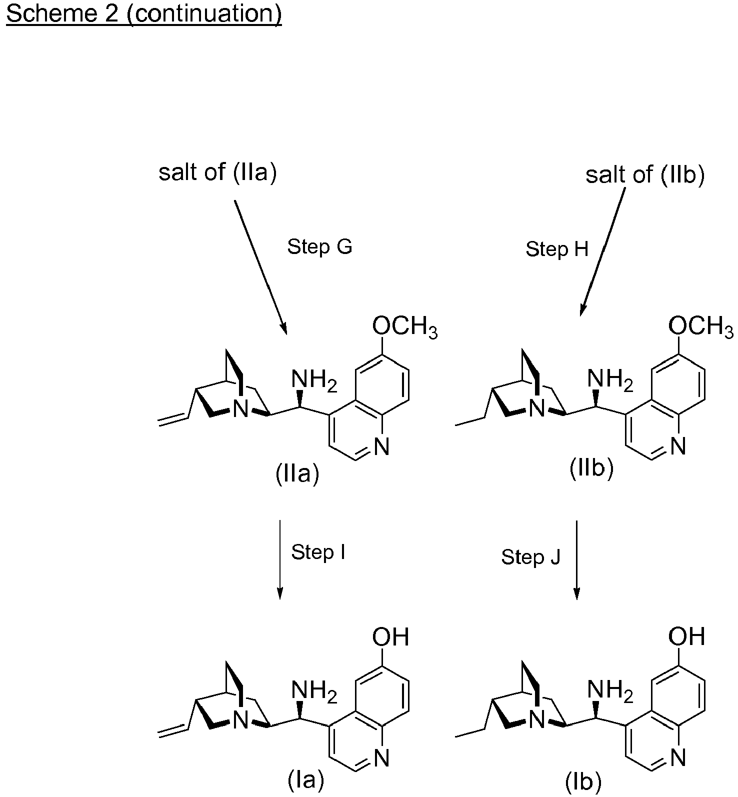

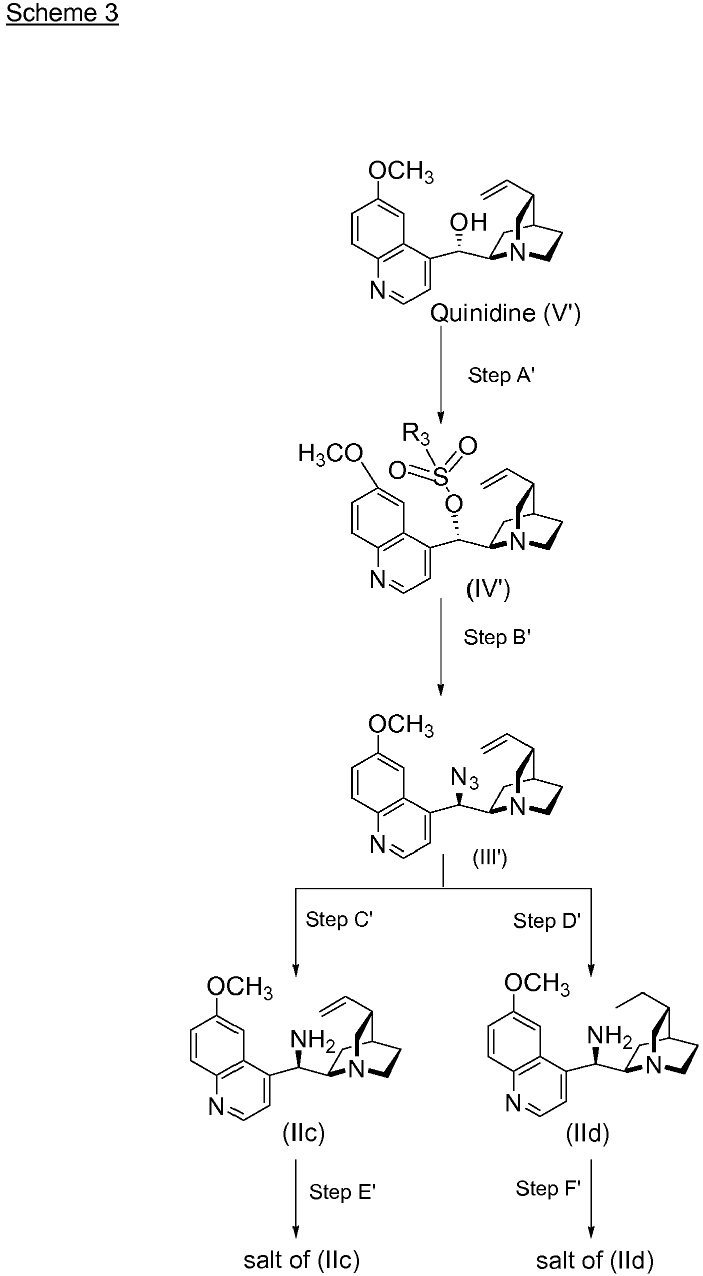

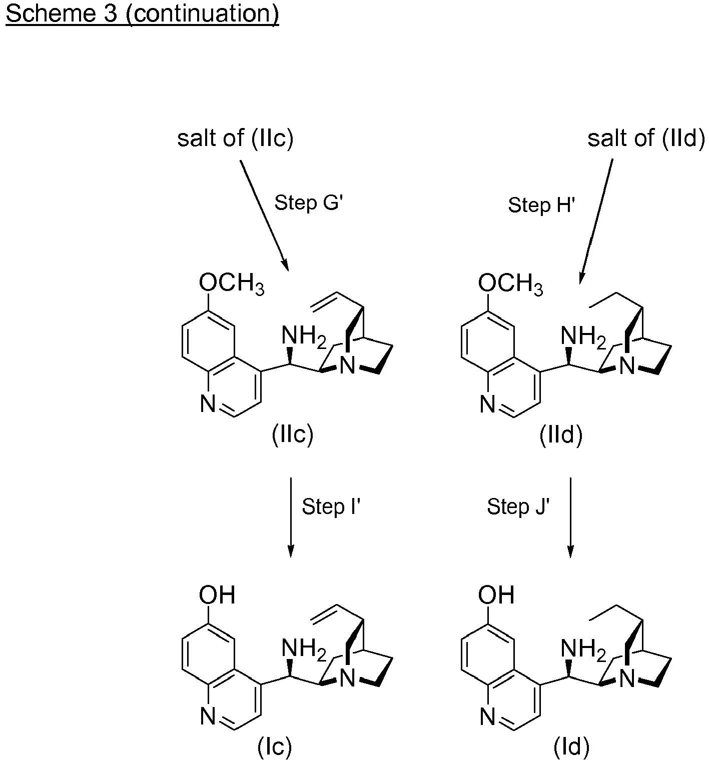

Hereinafter, a description of the preparation process by each step will be given in detail.

### Steps A and A': Here either quinine compound (V) or quinidine compound (V') reacts with a compound of formula (VI)

R₃-SO₂-Z (VI)

wherein Z is selected from an halide radical and a radical of formula -OSO₂R₄; and R₃ and R₄ are the same or different and are selected from the group consisting of (C₁-C₄)alkyl radical, (C₁-C₄)haloalkyl radical, and a phenyl radical optionally substituted by (C₁-C₄)alkyl;
in the presence of an aprotic nitrogenated base and a suitable solvent, to yield the compounds of formula (IV) and (IV').

In the present invention, the term "aprotic nitrogenated base" has to be understood as any nitrogen-containing organic compound that has the chemical properties of a base which, in addition, lacks an acidic hydrogen and does not display hydrogen bonding.

In one embodiment, the aprotic nitrogenated base is selected from the group consisting of tertiary amine, pyridine, 4-N,N-dimethylaminopyridine, and 1,4-diazabicyclo[2.2.2]octane. In a further embodiment, the aprotic nitrogenated base is a tertiary amine. Particularly, the tertiary amine used in steps A and A' is selected from: triethylamine, tributylamine, tripropylamine, N,N-diisopropylethylamine, and N,N,N',N'-tetramethylethane-1,2-diamine. Preferably, the tertiary amine is triethylamine.

In another embodiment, the aprotic nitrogenated base is selected from the group consisting of: triethylamine, tributylamine, tripropylamine, N,N-diisopropylethylamine, and N,N,N',N'-tetramethylethane-1,2-diamine, pyridine, 4-N,N-dimethylaminopyridine, and 1,4-diazabicyclo[2.2.2]octane.

In another embodiment, the compound of formula (VI) is one wherein Z means halide. In a further embodiment, the sulfonyl halide is selected from toluenesulfonyl, benzenesulfonyl, and methanesulfonyl halides. In another embodiment, the sulfonyl halide of formula (VI) is one wherein Z means chloride. In still another embodiment, the sulfonyl halide is selected from toluenesulfonyl chloride, benzenesulfonyl chloride, or methanesulfonyl chloride. Preferably, the sulfonyl halide is methanesulfonyl chloride.

The suitable solvent used in steps A and A' is an aprotic solvent. In one embodiment, the aprotic solvent is anhydrous. In another embodiment, the aprotic solvent is selected from tetrahydrofuran, 2-methyltetrahydrofuran, 1,4-dioxane, diethyl ether, *tert*-butylmethylether, acetone, ethyl acetate, chloroform and dichloromethane. Preferably, the suitable solvent is tetrahydrofuran. Even more preferably, the suitable solvent is anhydrous tetrahydrofuran.

The reaction is carried out at a temperature comprised of from 0 to 50 °C, generally at room temperature. In an embodiment, the addition of the compound of formula (VI) to the reaction mixture is carried out at 0 °C.

This step can also be carried out as disclosed in M. H. Franz et al., 2004.

Steps B and B': Here the resulting compounds of formula (IV) and (IV') are converted in the corresponding azides of formula (III) and (III'), respectively. Said steps are performed in the presence of a source of an azide moiety and a suitable solvent.

The source of azide moiety used in steps B and B' is selected from alkaline metal azides, hydrazoic acid and trimethylsilylazide. Particularly, the source of the azide moiety is sodium azide. When the source of the azide moiety is hydrazoic acid, step B or step B' further comprises a base, such as an inorganic base, allowing for the formation of the azide anion.

In one embodiment, the suitable solvent is a polar aprotic solvent. In another embodiment, said suitable solvent is selected from: dimethylformamide (DMF), dimethylsulfoxide (DMSO), methylpyrrolidone (NMP), hexamethylphosphoramide (HMPA), 1,3-Dimethyl-3,4,5,6-tetrahydro-2(1H)-pyrimidinone (DMPU), N-methylimidazole, 1,1,3,3-tetramethylurea (TMU), 1,3-Dimethyl-2-imidazolidinone (DMI), and mixtures thereof. Preferably, the suitable solvent is dimethylformamide.

The reaction is carried out under mild conditions, generally at a temperature comprised of from 45 to 80°C, and more particularly at about 65°C.

In contrast to the drawbacks associated to the conditions of Mitsunobu reaction (the requirement of expensive reagents, and the production of great amounts of undesired by-products), the use of an azide anion source allows for the generation of fewer residues, since lighter reagents are being used.

This step can also be carried out as disclosed in K. Kacprzak et al., 2010.

Steps C and C': here, the resulting compound of formula (III) or (III') is reduced in the presence of a metal hydride selected from lithium aluminium hydride, diisobutylaluminium hydride (DIBAL), Red-Al, borane, borane-diethyletherate and borane-tetrahydrofurane.

The suitable solvent is, preferably, a polar aprotic solvent. In one embodiment, the suitable solvent is anhydrous. In another embodiment, the suitable solvent is selected from the group consisting of: tetrahydrofuran, 1,4-dioxane, 2-methyltetrahydrofuran, *tert*-butylmethylether, and diethyl ether. Preferably, the suitable solvent is tetrahydrofuran, more preferably anhydrous tetrahydrofuran.

The reaction is carried out at low temperature, generally at a temperature comprised of from -10 to 65 °C. In one embodiment, the reaction is carried out at a temperature comprised of from -10 to 25°C. Preferably, the reaction is carried out at 0°C.

This step can also be carried out as disclosed in JP2010024173A.

Steps D and D': here, the resulting compound of formula (III) or (III') is reduced in the presence of a suitable catalyst, H₂, and a (C₁-C₁₀) aliphatic alcohol.

In one embodiment, the suitable catalyst is selected from the group consisting of Pd/C, Pd/Al₂O₃, Pd/CaCO₃, Ni Raney, Pd(OH)₂/C and PtO₂. Preferably, the catalyst is Pd/C.

In another embodiment, the (C₁-C₁₀)aliphatic alcohol is selected from the group consisting of: methanol, ethanol, and isopropanol. Preferably, the (C₁-C₁₀)aliphatic alcohol is methanol.

Steps E, E', F, and F': here the resulting compounds of formula (IIa), (IIb), (IIc), and (IId) are converted in the corresponding salts.

The conversion of compounds (IIa), (IIb), (IIc), and (IId) takes place in the presence of a solution of an acid and a solvent selected from acetonitrile and a (C₁-C₁₀) aliphatic alcohol.

In one embodiment, the (C₁-C₁₀)aliphatic alcohol is selected from methanol, ethanol, propanol, isopropanol, tert-butanol, and mixtures thereof.

In another embodiment, the acid is an halide acid. Preferably, the acid is chlorhydric acid (HCl). The acid can be added to a solution of the compound of formula (IIa), (IIb), (IIc) or (IId) as a solution or as a gas. In one embodiment, when the acid is added as a solution, it is dissolved in a solvent selected from the group consisting of 1,4-dioxane, diethyl ether, methanol, ethanol, and isopropanol. In another embodiment, when the acid is added as a gas, it can be bubbled through a solution of the compound of formula (IIa), (IIb), (IIc) or (IId).

In another embodiment, the solution of the acid has a concentration ranging from 1 to 7M.

In one embodiment, the conversion of the compound of formula (IIa), (IIc) or (IId) in the corresponding solid salt is performed by adding a solution of HCl in a solvent selected from the group consisting of: 1,4-dioxane, diethyl ether, methanol, ethanol, and isopropanol, to a solution of the compound (IIa), (IIc) or (IId) in a solvent selected from methanol, ethanol, propanol, isopropanol, and *tert*-butanol, followed by the addition of an anti-solvent such as ethyl acetate to provoke the precipitation of the corresponding solid salt. The solid salt thus obtained can then subsequently be filtered and washed to allow for its isolation.

The term "anti-solvent" is to be understood as any solvent in which the solid salt is insoluble. The skilled person in the art, considering the physicochemical properties of the solid salts is able to select the appropiate anti-solvent. Illustrative non-limitative examples of anti-solvents are ethyl acetate, acetonitrile, tetrahydrofurane, diethyl ether, methyl t-butyl ether, acetone, and butanone, among others

In another embodiment, the conversion of the compound of formula (IIb) in the corresponding solid salt is performed by adding a solution of HCl in 1,4-dioxane or isopropanol to a solution of the compound (IIb) in acetonitrile. The precipitate thus obtained can be filtered and washed to allow for the obtention of the solid salt of (IIb).

Steps G, G', H, and H': here the resulting compounds salts of (IIa), (IIb), (IIc), and (IId), are subjected to aqueous basic conditions in the presence of an organic solvent that is inmiscible with water, in order to get the corresponding compounds of formula (IIa)-(IId) in the organic phase.

The base to be used can be any water soluble inorganic base selected from the group consisting of ammonium, and alkaline or alkaline earth salts of hydroxide and carbonate. Thus, in one embodiment, the base is selected from the group consisting of NaOH, KOH, LiOH, Cs₂CO₃, Na₂CO₃, K₂CO₃, and NH₄OH. Preferably, the base is NH₄OH.

Steps I, I', J, and J': here, the resulting compounds of formula (IIa)-(IId) are demethylated in order to obtain the corresponding compound of formula (Ia)-(Id).

The demethylation step, as it has been deeply explained above, is carried out in the presence of a thiolate salt, and a polar aprotic solvent, in mild conditions.

From the above, it can be concluded that the configuration of the stereogenic centers, other than C9, in the compounds of formula (II) (namely (IIa), (IIb), (IIc), and (IId)) is due to the stereoselective transformations from quinine (V) and quinidine (V').

In a further step, the resulting compounds of formula (Ia)-(Id) can be purified. In one embodiment, the compounds of formula (Ia)-(Id) are purified by elution through an ion exchange column. In another embodiment, the compounds of formula (Ia)-(Id) are purified by column chromatography. Alternatively, the compounds of formula (Ia)-(Id) can be purified by precipitation of the pure compounds (Ia)-(Id), or a salt or a co-crystal thereof, followed by filtration and washing.

The most adequate conditions for carrying out the process described herein vary depending on the parameters considered by an expert in the art, such as the concentration, temperature, the solvent used during the reaction or the isolation of the products, and the like. Such adequate conditions can be readily determined by said skilled person in the art with the help of the teachings of the examples given in this description.

It is also part of the present invention the provision of single reaction steps of the global process to obtain compounds of formula (IIa)-(IId), as well as the combination of two or more sequential steps of the global process described above, the process being able to be carried out in sequential steps isolating the intermediates obtained.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES:

### Example 1: Preparation of compounds of formula (IV and IV')

Quinine (V) or quinidine (V') (5.0 g; 15.4 mmol) was weighed in a 3-necked 250 ml round bottomed flask under nitrogen atmosphere and subsequently dissolved in dry tetrahydrofuran (THF) (23 ml). Triethylamine (8.60 ml; 61.7 mmol) was added all at once at room temperature and the mixture was magnetically stirred. The flask was placed in a water-ice bath so that the internal temperature was stabilized around 0-4 °C. After 10 min methanesulfonyl chloride (2.86 ml; 37.0 mmol) dissolved in dry THF (9 ml) was added dropwise with an addition funnel (30 min addition). Additional dry THF (3 ml) was used to rinse the entire reagent inside. After 4 h, conversion was complete.

The reaction was quenched at 0-4 °C with the slow addition (addition funnel) of an aqueous solution of NaHCO₃ (3.5 % w/w) (35 ml). Two transparent yellow phases were obtained and subsequently separated in a separation funnel. The organic phase was reserved and the aqueous phase was extracted two times with ethyl acetate (2 x 10 ml). All organic layers were combined and transferred to the separation funnel. Newly formed aqueous layer was removed and the organic layer was concentrated under reduced pressure. Water was removed with the help of ethyl acetate (ethyl acetate added twice 2 x 10 ml when about 10 ml solution are left) at 110 mbar and 35 °C bath. All solvents were removed under high vacuum. Crude compounds of formula (IV) (if starting from quinine (V)) and (IV') (if starting from quinidine (V')) were obtained as yellow sticky oils that may solidify on standing providing white to off-white solids.

The crude was submitted to the next reaction.
Compound of formula (IV): white solid, ¹H NMR (500 MHz, CDCl₃) δ 8.79 (d, 1H, *J*= 4.5 Hz), 8.04 (d, 1H, *J* = 9.2 Hz), 7.44 (bs, 1H), 7.40 (dd, 1H, *J*₁ = 9.2, *J*₂ = 2.6 Hz), 7.36 (bs, 1H), 6.17 (bs, 1H), 5.82 (ddd, 1H, *J*₁ = 17.4, *J*₃ = 10.0, *J*₃ = 7.2 Hz), 5.04 - 4.96 (m, 2H), 3.95 (s, 3H), 3.39 (bs, 1H), 3.16 - 3.03 (m, 1H), 3.03 - 2.88 (m, 1H), 2.70 - 2.44 (m, 5H), 2.33 - 2.17 (m, 1H), 2.15 - 2.00 (m, 1H), 1.95 - 1.86 (m, 1H), 1.83 - 1.69 (m, 1H), 1.70 - 1.59 (m, 1H) and 1.59 - 1.43 (m, 1H). MS: 403 (M+H).

Compound of formula (IV'): white solid, ¹H NMR (400 MHz, CDCl₃) δ 8.79 (d, 1H, *J* = 4.5 Hz), 8.05 (d, 1H, *J* = 9.3 Hz), 7.45 (bs, 1H), 7.41 (dd, 1H, *J₁* =9.2, *J₂* = 2.7 Hz), 7.33 (bs, 1H), 6.21 (bs, 1H), 6.03 (ddd, 1H, *J₁* =17.1, *J₂* = 10.5, *J₃*= 7.3 Hz), 5.13 (dt, 1H, *J_{d}*=10.5, *Jₜ* = 1.4 Hz), 5.11 (dt, 1H, *J_{d}* =17.0, *Jₜ =* 1.5 Hz), 3.96 (s, 3H), 3.32 (bs, 1H), 2.89 (d, 2H, *J* = 8.9 Hz), 2.82 - 2.55 (m, 4H), 2.35 - 2.20 (m, 1H), 2.17 - 2.01 (m, 1H), 2.01 - 1.90 (m, 1H), 1.89 - 1.82 (m, 1H), 1.68 (m, 1H) and 1.61 - 1.50 (m, 2H). MS: 403 (M+H).

### Example 2: Preparation of compounds of formula (III and III')

Sodium azide (1.5 g, 23.1 mmol) was weighed and suspended in dry N,N-dimethylformamide (DMF) (10 ml) in a 3-necked 250 ml round bottomed flask under nitrogen atmosphere. The suspension was magnetically stirred. Crude and dry compound of formula (IV) from previous reaction (assumed 15.4 mmol) was dissolved in dry DMF (15 ml) and added all at once onto the suspension of sodium azide. Additional dry DMF (5 ml) was used to rinse all crude compound of formula (IV) inside the reaction flask. The mixture was heated so that the internal temperature is 65 °C and a refrigerant was conneted to the reaction flask. After 16 h stirring at 65 °C the reaction was complete.

The mixture was cooled in a water-ice bath so that the internal temperature was 0-4 °C. When the temperature was stabilized, the reaction was quenched with the slow addition (via addition funnel) of 1 M aqueous solution of sodium hydroxide (30 ml). The addition took 30 min. The resulting mixture was a yellow solution with insoluble yellow thick oil. The mixture was transferred to a separation funnel and extracted with *tert* butyl methyl ether (MTBE, 3 x 10 ml). The organic layers were combined,washed with 1 M aqueous solution of NaOH (10 ml) and transferred to a 100 ml round bottomed flask, where the solvents were removed under reduced pressure. The crude product was dried under high vacuum. Crude compound of formula (III) was obtained as a yellow oil.

The crude (5.2 g) is submitted to the next reaction.

Analogously, crude compound of formula (III') was obtained as a yellow oil following the same procedure as for crude compound of formula (III) but starting from crude compound of formula (IV') instead of (IV).

Compound of formula (III): yellow oil, ¹H NMR (500 MHz, CDCl₃) δ 8.79 (d, 1H, *J* = 4.5 Hz), 8.08 (d, 1H, *J* = 9.2 Hz), 7.47 (bs, 1H), 7.43 (dd, 1H, *J₁* =9.2, *J₂* = 2.7 Hz), 7.34 (d, 1H, *J* = 4.5 Hz), 5.76 (ddd, 1H, *J₁* = 17.4, *J₂* = 10.4, *J₃* = 7.3 Hz), 5.06 - 4.93 (m, 3H), 3.98 (s, 3H), 3.43 - 3.27 (m, 2H), 3.26 - 3.16 (m, 1H), 2.98 - 2.88 (m, 1H), 2.88 - 2.78 (m, 1H), 2.40 - 2.23 (m, 1H), 1.73 - 1.65 (m, 1H), 1.65 - 1.49 (m, 2H), 1.47 - 1.34 (m, 1H) and 0.85 - 0.70 (m, 1H). MS: 350 (M+H).

Compound of formula (III'): yellow oil, ¹H NMR (500 MHz, CDCl₃) δ 8.77 (d, 1H, *J* = 4.5 Hz), 8.05 (d, 1H, *J* = 9.2 Hz), 7.43 (bs, 1H), 7.41 (dd, 1H, *J₁* = 9.1, *J₂* = 2.7 Hz), 7.35 (d, 1H, *J* = 4.5 Hz), 5.86 (ddd, 1H, *J₁* =17.0, *J₂* = 11.0, *J₃* = 6.4 Hz), 5.12 - 5.02 (m, 3H), 3.96 (s, 3H), 3.38 - 3.19 (m, 1H), 3.19 - 3.09 (m, 1 H), 3.09 - 2.82 (m, 3H), 2.37 - 2.22 (m, 1H), 1.71 - 1.61 (m, 1H), 1.61 - 1.42 (m, 2H), 1.18 - 1.05 (m, 1H) and 1.01 - 0.84 (m, 1H). MS: 350 (M+H).

### Example 3: Preparation of compounds of formula (IIa) and (IIc)

Crude compound of formula (III) from previous reaction (assumed 15.4 mmol) was dissolved in anhydrous THF (75 ml) in a 3-necked 250 ml round bottomed flask under nitrogen atmosphere. The mixture was magnetically stirred and cooled down in a water-ice bath so that the internal temperature was 0-4 °C. When the temperature was stabilized, lithium aluminium hydride solution in THF (2.4 M) (3.9 ml, 9.4 mmol) was added slowly via syringe through a rubber septum. A typical addition time is 1 h. Upon addition the colour of the reaction mixture turns first to orange and then to red. Gas evolution could be observed. The reaction was stirred at 0 °C for 4 hours.

When the reaction was complete, ethyl acetate (25 ml) was added slowly to quench the reaction while stirring at 0-4 °C. An aqueous solution of NH₄OH (0.5 M) (60 ml) was then added dropwise with an addition funnel. The aqueous and the organic layers were separated using a separation funnel. The aqueous layer was further extracted with ethyl acetate (2 x 20 ml). The organic layers were combined and transferred to a separation funnel to remove the newly formed aqueous layer. The solvents were removed under reduced pressure evaporator. Residual water was removed by adding ethyl acetate (10 ml) and submitting the mixture to reduced pressure (2 times).

Crude compound of formula (IIa) was obtained as a yellow thick oil which was finally dried under high vacuum.

Analogously, crude compound of formula (IIc) was obtained as a yellow thick oil following the same procedure as for crude compound of formula (IIa) but starting from crude compound of formula (III') instead of (III).

Compound of formula (IIa): yellow oil, ¹H NMR (400 MHz, CDCl₃): δ 8.73 (d, 1H, *J* = 4.5 Hz), 8.01 (d, 1H, *J* = 9.4 Hz), 7.64 (bs, 1H), 7.46-7.41 (m, 1H), 7.36 (dd, 1H, *J₁* = 9.3, *J₂* = 2.8 Hz), 5.78 (ddd, 1H, *J₁* = 17.3, *J₂* = 10.1, *J₃* = 7.5 Hz), 5.01-4.90 (m, 2H), 4.67-4.51 (m, 1H), 3.94 (s, 3H), 3.26 (dd, 1H, *J₁* = 13.7, *J₂* = 10.0 Hz), 3.23-3.15 (m, 1H), 3.12-2.98 (m, 1H), 2.84-2.74 (m, 2H), 2.31-2.23 (m, 1H), 2.12-1.93 (m, 2H) 1.64-1.59 (m, 1H), 1.58-1.48 (m, 2H), 1.45-1.36 (m, 1H) and 0.79-0.71 (m, 1H). ¹³C {¹H} NMR (125 MHz, CDCl₃, 213 K): δ 157.3, 148.0, 146.8, 143.9, 141.6, 131.6, 119.4, 114.5, 100.2, 61.9, 55.5, 55.5, 50.3, 40.7, 39.6, 27.6, 27.0 and 25.6. MS: 324 (M+H).

Compound of formula (IIc): yellow oil, ¹H NMR (400 MHz, CDCl₃): δ 8.74 (d, 1H, *J* = 4.5 Hz), 8.02 (d, 1H, *J* = 9.2 Hz), 7.73-7.49 (m, 2H), 7.37 (dd, 1H, *J₁* = 9.4, *J₂* = 2.8 Hz), 5.88 (ddd, 1H, *J₁* = 17.1, *J₂* = 10.6, *J₃* = 6.6 Hz), 5.08 (dt, 1H, *J_{d} =* 8.8, *Jₜ* = 1.6 Hz), 5.06-5.03 (m, 1H), 4.75-4.60 (m, 1H), 3.96 (s, 3H), 3.11-2.88 (m, 5H), 2.34-2.22 (m, 1H), 2.15-1.94 (m, 2H), 1.64-1.59 (m, 1H), 1.58-1.49 (m, 2H), 1.17-1.09 (m, 1H) and 1.00-0.90 (m, 1H). ¹³C {¹H} NMR (125 MHz, CDCl₃, 213 K): δ 157.2, 147.9, 147.5, 143.8, 131.4, 128.4, 122.1, 119.6, 114.4, 99.5, 62.6, 55.4, 49.1, 48.9, 46.6, 38.7, 26.8, 25.9 and 24.2. MS: 324 (M+H).

### Example 4: Preparation of compounds of formula (IIb) and (IId)

Crude compound of formula (III) from Example 2 (assumed 15.4 mmol) was dissolved in methanol (10 ml) and the solution was transferred to a high pressure reactor (25 ml) with a cross-shape teflon coated stirring bar. Once palladium on carbon (5 % w/w) (500 mg) was added to the solution, the reactor was sealed, purged three times with hydrogen and finally charged with hydrogen (20 bar). After 2 h stirring at room temperature, the reactor was purged three times with hydrogen and charged again with hydrogen (20 bar). After 24 h the reactor was depressurized and the reaction mixture was filtered through a pad of packed celite in ethyl acetate. The celite was washed with ethyl acetate until the filtrating solution exhibited no colour. The solvents of the filtrate were removed under reduced pressure and the crude was dried under vacuum to yield the crude product of formula (IIb) as a yellow thick oil which was finally dried under high vacuum.

Analogously, crude product of formula (IId) was obtained as a yellow thick oil following the same procedure as for compound of formula (IIb) but starting from crude compound of formula (III') instead of (III).

Compound of formula (IIb): yellow oil, ¹H NMR (500 MHz, CDCl₃) δ 8.71 (d, 1H, *J* = 4.6 Hz), 8.00 (d, 1H, *J* = 9.2 Hz), 7.62 (bs, 1H), 7.50 - 7.38 (m, 1H), 7.35 (dd, 1H, *J₁* = 9.1, *J₂* = 2.6 Hz), 4.63 - 4.46 (m, 1H), 3.93 (d, 3H, *J* = 1.2 Hz), 3.21 (dd, 1H, *J₁* = 13.7, *J₂* = 9.9 Hz), 3.18 - 3.10 (m, 1H), 3.08 - 2.95 (m, 1H), 2.74 (ddd, 1H, *J₁* = 14.7, *J₂* = 10.4, *J₃*= 4.7 Hz), 2.52 - 2.43 (m, 1H), 2.11 (bs, 3H), 1.62 - 1.15 (m, 7H), 0.78 (t, 3H, *J* = 7.3 Hz) and 0.75 - 0.66 (m, 1H). ¹³C {¹H} NMR (125 MHz, CDCl₃, 213 K) δ 157.1, 147.8, 146.8, 143.7, 131.3, 128.4, 121.3, 119.2, 100.0, 61.6, 57.0, 55.3, 50.1, 40.7, 36.3, 28.0, 27.2, 25.1, 24.3 and 12.0. MS: 326 (M+H).

Compound of formula (11d): yellow oil, ¹H NMR (500 MHz, CDCl₃) δ 8.73 (d, 1H, *J* = 4.5 Hz), 8.02 (d, 1H, *J* = 9.2 Hz), 7.63 (bs, 1H), 7.55 - 7.45 (m, 1H), 7.37 (dd, 1H, *J₁* = 9.3, *J₂* = 2.7 Hz), 4.77 - 4.53 (m, 1H), 3.95 (s, 3H), 3.08 - 2.84 (m, 4H), 2.62 (ddd, 1H, *J₁* = 14.1, *J₂* = 7.3, *J₃* = 2.3 Hz), 2.07 (bs, 2H), 1.64 - 1.26 (m, 7H), 1.12 - 1.00 (m, 1H), 0.98 - 0.91 (m, 1H), and 0.86 (t, 3H, *J* = 7.2 Hz) .¹³C{¹H} (125 MHz, CDCl₃)δ 157.0,147.8, 147.4, 143.7,131.4, 128.4, 121.8, 119.5, 99.5, 62.6, 55.2, 49.0, 48.9, 48.9, 36.5, 26.7, 25.6, 25.0, 23.9 and 11.9. MS: 326 (M+H).

### Example 5: Preparation of (IIa)·3 HCl

The crude from compound of formula (IIa) for 15.4 mmol of starting quinine, was dissolved in methanol (18 ml) and hydrogen chloride solution in 2-propanol (5-6 M) (6.7 ml) was added via syringe. Upon addition the solution turned from yellow to red. The mixture was heated to 55 °C and, after 5 minutes ethyl acetate (18 ml) was added slowly. Upon addition some white clouding formed and got redissolved. At the end of the addition a dark orange solution was obtained. The mixture was allowed to reach room temperature while stirring. After 2 h a pale yellow precipitate had formed. The mixture was stirred 1 h longer in a water-ice bath. The solid was filtered, washed with ethyl acetate/methanol (1:1), and dried under vacuum overnight. The product was obtained as a pale yellow solid (3.4 g, 50 % yield relative to starting quinine).

Compound of formula (II)·3 HCl: pale yellow solid; ¹H NMR (500 MHz, D₂O) δ 9.04 (d, 1H, *J* = 5.8 Hz), 8.30 (d, 1H, *J* = 9.3 Hz), 8.16 (d, 1H, *J* = 5.7 Hz), 7.94 (dd, 1H, *J₁* = 9.3, J₂ = 2.5 Hz), 7.84 (d, 1H, *J* = 2.5 Hz), 5.91 (ddd, 1H, *J₁* =17.2, *J₂* = 10.6, *J₃* = 6.4 Hz), 5.55 (d, 1H, *J* = 10.8 Hz), 5.27 (ddd, 1H, *J₁* =17.3, *J₂*= 1.5, *J₃*= 1.0 Hz), 5.25 (ddd, 1H, *J₁*=10.6, *J2*=1.5, *J3*=1.0Hz, 1H), 4.37 - 4.24 (m, 1H), 4.13 (s, 3H), 4.04 - 3.94 (m, 1H), 3.85 (dd, 1H, *J₁* = 13.3, *J₂*= 10.5 Hz), 3.64 - 3.43 (m, 2H), 3.03 - 2.88 (m, 1H), 2.21 - 2.00 (m, 3H), 1.99 - 1.84 (m, 1H) and 1.24 - 1.12 (m, 1H). ¹³C {¹H} NMR (125 MHz, D₂O) δ 161.4, 146.8, 141.0, 137.1, 134.5, 129.6, 128.4, 123.7, 121.1 (broad), 116.9, 102.7, 59.3, 56.7, 53.9, 48.8 (broad), 42.5, 35.6, 25.3, 23.5 and 23.2.

### Example 6: Preparation of (IIb)·HCl

The crude from compound of formula (IIb) for 15.4 mmol of starting quinine, was dissolved in acetonitrile (19 ml) and heated to 55 °C. Hydrogen chloride solution in 1,4-dioxane (4 M) (2.8 ml) was then added via syringe. With the first drops some white gummy precipitate formed in the yellow solution but rapidly equilibrated to a white powder after a few seconds stirring. The suspension was stirred at 55 °C for 5 minutes longer and then was allowed the mixture to reach room temperature and stirred for one hour before collecting the white solid by filtration. The solid was washed with acetonitrile and dried under vacuum overnight.

Compound of formula (IIb)·HCl: white solid, ¹H NMR (500 MHz, D₂O) δ 8.71 (d, 1H, *J* = 4.7 Hz), 7.98 (d, 1H, *J* = 9.5 Hz), 7.68 - 7.22 (m, 3H), 4.01 (s, 3H), 3.94 - 3.77 (m, 2H), 3.72 (dd, 1H, *J₁* = 12.9, *J₂*= 10.5 Hz), 3.52 - 3.24 (m, 1H), 3.03 (ddd, 1H, *J₁* =12.8, *J₂*= 5.9, *J₃*= 2.6 Hz), 2.07 - 1.90 (m, 3H), 1.91 - 1.83 (m, 1H), 1.82 - 1.68 (m, 1H), 1.52 - 1.29 (m, 2H), 1.11 - 0.91 (m, 1H) and 0.85 (t, 3H, *J* = 7.4 Hz). ¹³C {¹H} NMR (125 MHz, D₂O) δ 157.7, 147.4, 146.2 (broad), 142.9, 130.0, 127.2, 121.8, 118.5 (broad), 101.2, 61.5, 55.7, 55.3, 49.5 (broad), 40.9, 34.2, 25.8, 24.0, 23.7, 23.3 and 10.7.

### Example 7: Preparation of compounds of formula (IIc)·3 HCl and (IId)·3HCl

The crude from compound of formula (IIc) (or (IId)) for 15.4 mmol of starting quinidine, was dissolved in methanol (110 ml) and the mixture was heated to 55 °C. Hydrogen chloride solution in 2-propanol (5-6 M) (6.7 ml) was added via syringe and the colour of the solution turned to red. The mixture was stirred at 55 °C for 1 h longer and then was allowed to reach room temperature while stirring. A light white precipitate formed. Ethyl acetate (15 ml) was then added and the mixture was stirred 1 h longer in an ice bath. The solid was filtered, washed with a methanol/ethyl acetate 1/1 mixture, and dried under vacuum overnight. The product was obtained as a white solid (3.8 g, 56 % yield relative to starting quinidine).

Compound of formula (IIc)·3 HCl: white solid, ¹H NMR (500 MHz, D₂O) δ 9.07 (d, 1H, *J* = 5.8 Hz), 8.29 (d, 1H, *J* = 9.4 Hz), 8.22 (d, 1H, *J* = 5.7 Hz), 7.93 (dd, 1H, *J₁* =9.4, *J₂* = 2.5 Hz), 7.78 (d, 1H, *J* = 2.5 Hz), 5.89 (ddd, 1H, *J₁* =17.4, *J₂* = 10.8, *J₃* = 5.4 Hz), 5.67 (d, 1H, *J* = 10.6 Hz), 5.32 (ddd, 1H, *J₁* =10.5 Hz, *J₂*=1.5Hz, *J3*=0_{.}5Hz), 5.31 (ddd, 1H, *J₁*=17.5Hz, J2=1.5Hz, J3=0.5Hz,), 4.35 (q, 1H, *J* = 9.6 Hz), 4.13 (s, 3H), 3.83 - 3.74 (m, 1H), 3.75 - 3.65 (m, 2H), 3.65 - 3.55 (m, 1H), 2.97 - 2.88 (m, 1H), 2.14 - 2.05 (m, 2H), 2.05 - 1.95 (m, 1H), 1.59 - 1.49 (m, 1H) and 1.41 - 1.33 (m, 1H). ¹³C {¹H} NMR (125 MHz, D₂O) δ 161.4, 146.6, 141.0, 136.3, 134.5, 129.6, 128.7, 123.7, 121.1, 116.9, 102.0, 59.8, 56.8, 49.6, 48.1 (broad), 46.9, 35.1, 25.1, 23.0 and 21.8.

Compound of formula (IId)·3 HCl: white solid, ¹H NMR (500 MHz, D₂O) δ 9.09 (d, 1H, *J* = 5.7 Hz), 8.31 (d, 1H, *J* = 9.4 Hz), 8.24 (d, 1H, *J* = 5.8 Hz), 7.94 (dd, 1H, *J* = 9.3, 2.5 Hz), 7.84 (d, 1H, *J* = 2.5 Hz), 5.74 (d, 1H, *J* = 10.2 Hz), 4.41 - 4.29 (m, 1H), 4.13 (s, 3H), 3.76 (dd, 1H, *J₁* = 13.5, *J₂*= 10.3 Hz), 3.71 - 3.63 (m, 1H), 3.63 - 3.51 (m, 1H), 3.39 (ddd, 1H, *J₁* = 13.5, *J₂*= 8.6, *J₃*= 2.5 Hz), 2.18 - 2.03 (m, 1H), 2.03 - 1.91 (m, 3H), 1.60 - 1.46 (m, 2H), 1.46 - 1.30 (m, 2H) and 0.91 (t, 3H, *J* = 7.4 Hz). ¹³C {¹H} NMR (100 MHz, D₂O) δ 161.5, 146.6, 141.0, 134.6, 129.6, 128.7, 123.8, 121.1 (broad), 102.0, 59.8, 56.8, 49.5, 49.2, 48.1 (broad), 34.1, 24.5, 23.7, 22.7, 22.4 and 10.5.

### Example 8: Formation of the free amine from the hydrochlorides of formula (IIa)·3HCl, (IIb)·HCl, (IIc)·3HCl, and (IId)·3HCl

Salt (hydrochloride or trihydrochloride) was weighed (3.0 g) and suspended in dichloromethane (20 ml). 5 M aqueous solution of NH₄OH (15 ml) was slowly added while stirring. After five minutes stirring the biphasic mixture was transferred to a separation funnel and the organic layer was reserved. The aqueous layer was washed with dichloromethane (2 x 15 ml). The organic layers were combined, dried over sodium sulphate, filtered and the solvent was removed under reduced pressure. The product, thick oil, was dried under high vacuum.

¹H and ¹³C {¹H} NMR NMR data of the products have been collected above, under Examples 3 and 4.

### Example 9: Preparation of the compounds of formula (Ia)-(Id)

Free amine of formula (IIa) (1.5 mmol) obtained in the previous example was weighed out in a 10 ml round bottomed flask under nitrogen atmosphere. Sodium ethanethiolate (8.8 mmol) was added all at once followed by dimethyl sulfoxide (1.5 ml). The mixture was heated to 80 °C and stirred for 72 h at that temperature. Once the reaction was finished, the mixture was allowed to reach room temperature, diluted with water (10 ml) and washed with dichloromethane (3 x 5 ml). Saturated aqueous solution of ammonium chloride was added dropwise to the aqueous layer. When the pH was about 9, the aqueous solution was extracted with dichloromethane (6 x 5 ml). The organic layers were combined, dried over sodium sulphate and filtered. The solvent was removed under reduced pressure and the crude was obtained as a red oil, which was purified by flash column chromatography with silica gel and a mixture of ethyl acetate, methanol and concentrated aqueous ammonium hydroxide (100/10/1) as eluent. The product was isolated as a pale orange solid (0.283 g, 59 % yield relative to starting (IIa)).

Crude product of formula (Ib) was obtained as a pale orange solid with a yield of 54 % following the same procedure as for compound of formula (Ia) but starting from the free amine of formula (IIb).

Crude product of formula (Ic) was obtained as a pale orange solid with a yield of 63 % following the same procedure as for compound of formula (Ia) but starting from the free amine of formula (IIc).

Crude product of formula (Id) was obtained as a pale orange solid with a yield of 61 % following the same procedure as for compound of formula (Ia) but starting from the free amine of formula (IId) and doubling the scale.

Compound of formula (Ia): pale orange solid, ¹H NMR (500 MHz, CD₃OD) δ 8.61 (d, 1H, *J* = 4.6 Hz), 7.91 (d, 1H, *J* = 9.1 Hz), 7.57 (bs, 1H), 7.54 (d, 1H, *J* = 4.6 Hz), 7.38 (dd, 1H, *J₁* = 9.1, J₂ = 2.5 Hz), 5.87 (ddd, 1H, *J₁* = 17.5, *J₂* = 10.4, *J₃* = 7.5 Hz), 5.05 (d, 1H, *J* = 17.1 Hz), 5.00 (d, 1H, *J* = 10.4 Hz), 4.71 - 4.58 (m, 1H), 3.36 - 3.26 (m, 2H), 3.26 - 3.15 (m, 1H), 2.94 - 2.80 (m, 2H), 2.44 - 2.30 (m, 1H), 1.73 - 1.52 (m, 3H), 1.52 - 1.36 (m, 1H) and 0.77 (dd, 1H, *J₁*= 13.6, *J₂*= 7.4 Hz). ¹³C {¹H} NMR (125 MHz, CD₃OD) δ 158.4, 148.2, 147.5, 144.2, 142.6, 131.5, 130.6, 123.7, 120.4 (broad), 115.1, 105.5 (broad), 63.2 (broad), 56.8, 41.7, 40.7, 28.8, 28.4 and 26.7.

Compound of formula (Ib): pale orange solid, ¹H NMR (500 MHz, CD₃OD) δ 8.61 (d, 1H, *J* = 4.6 Hz), 7.91 (d, 1H, *J* = 9.1 Hz), 7.60 - 7.52 (m, 2H), 7.38 (dd, 1H, *J₁* = 9.1, *J₂* = 2.6 Hz), 5.89 (ddd, 1H, *J₁* = 17.3, *J₂* = 10.4, *J₃* = 6.9 Hz), 5.10 (dt, 1H, *J_{d}* = 13.9, *Jₜ* = 1.6 Hz), 5.08 - 5.06 (m, 1H), 4.68 (d, 1H, *J* = 10.2 Hz), 3.25 - 2.91 (m, 5H), 2.34 (q, 1H, *J* = 8.5 Hz), 1.68 - 1.47 (m, 3H) and 1.13 - 0.95 (m, 2H). ¹³C{¹H} NMR (125 MHz, CD₃OD) δ 158.3, 148.3, 147.6, 144.2, 141.2, 131.5, 130.6, 123.7, 120.6 (broad), 115.6, 105.6 (broad), 63.4 (broad), 50.3, 48.1, 40.6, 29.0, 27.1 and 25.9.

Compound of formula (Ic): pale orange solid, ¹H NMR (400 MHz, CD₃OD) δ 8.61 (d, 1H, *J* = 4.7 Hz), 7.91 (d, 1H, *J* = 9.1 Hz), 7.61 - 7.50 (m, 2H), 7.37 (dd, 1H, *J₁* = 9.1, J₂= 2.6 Hz), 4.70 - 4.57 (m, 1H), 3.41 - 3.32 (m, 1H), 3.28 - 3.17 (m, 1H), 2.93 - 2.80 (m, 1H), 2.63 (ddd, 1H, J₁ = 13.5, *J₂*= 4.8, *J₃* = 2.4 Hz), 1.79 - 1.51 (m, 4H), 1.51 - 1.31 (m, 4H), 0.87 (t, 3H, *J* = 7.4 Hz) and 0.78 (dd, 1H, *J₁*= 13.5, *J₂*= 7.2 Hz). ¹³C {¹H} NMR (125 MHz, CD₃OD) δ 158.7, 148.1 (broad), 147.5, 144.2, 131.5, 130.4, 123.8, 123.7 (broad), 105.5 (broad), 63.1 (broad), 58.0, 41.8, 38.0, 28.3, 28.2, 26.3, 26.1 and 12.2.

Compound of formula (Id): pale orange solid, ¹H NMR (400 MHz, CD₃OD) δ 8.60 (d, 1H, J = 4.7 Hz), 7.90 (d, 1H, J = 9.1 Hz), 7.55 (d, 1H, J = 4.7 Hz), 7.52 (bs, 1H), 7.37 (dd, 1H, *J₁* = 9.1, *J₂* = 2.6 Hz), 4.67 (d, 1H, *J* = 10.2 Hz), 3.22 - 2.94 (m, 4H), 2.77 (dd, 1H, *J₁* = 12.8, *J₂* = 7.1 Hz), 1.71 - 1.49 (m, 4H), 1.49 - 1.27 (m, 2H), 1.08 - 0.98 (m, 2H) and 0.90 (t, 3H, *J* = 7.4 Hz). ¹³C {¹H} NMR (125 MHz, CD₃OD) δ 159.2, 148.2, 147.2, 144.1, 131.4, 130.7, 124.2, 120.4 (broad), 105.4 (broad), 63.6 (broad), 50.3, 49.9, 38.3, 27.7, 27.1, 26.6, 25.7 and 12.3.

Analogously, compounds of formula (Ia)-(Ib) were obtained replacing the dimethylsulfoxide by dimethylformamide, at a temperature of 110°C; as well as replacing the sodium ethanethiolate by sodium *t*-butylthiolate.

### Example 10: Compound of formula (Id) as catalyst

In order to determine whether the compound of formula (Id) is useful as catalyst, the following reaction was performed:

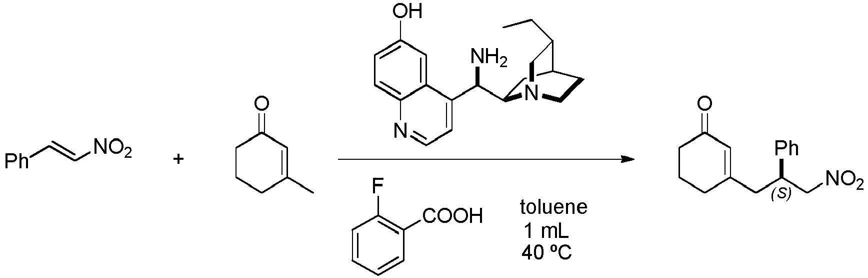

Briefly, compound of formula (Id) (0.02 mmol, 6.2 mg, 10 mol%) was weighed in an ordinary test tube equipped with a Teflon-coated stir bar.

2-fluoro-benzoic acid (0.04 mmol, 6.5 mg, 20 mol%) and *trans*-β-nitrostyrene (29.8 mg, 0.2 mmol) were added. Toluene (1 mL) was added to the mixture and was then stirred for 10 min. at room temperature. All components dissolved except for compound of formula (Id). 3-Methyl-2-cyclohexenone (45 µL, 0.4 mmol, 2.0 equiv.) were then added and the test tube was closed with a septum and immerged in an oil bath at 40° C. Stirring continued for 48 h at that temperature until the conversion was 83 % (¹H NMR). The crude mixture was diluted with dichloromethane (1 mL) and flushed through a plug of silica in a Pasteur pippette, using dichloromethane/ethyl acetate 1:1 as the eluent (5 mL). The solvent was removed under reduced pressure and the title compound was isolated as a white solid in 67% yield and 98% ee by flash column chromatography using hexane/ethyl acetate (9/1) as the eluent mixture.

HPLC analysis on a Daicel Chiralpak AD-H column: 85/15 hexane/*i*-PrOH, flow rate 0.75 mL/min, λ = 214, 230, 254 nm: *τmajor* = 15.7 min, *τminor* = 17.9 min; ¹H NMR (500 MHz, CDCl₃): δ 1.85-1.95 (m, 2H), 2.14-2.32 (m, 4H), 2.58-2.67 (m, 2H), 3.69-3.80 (m, 1H), 4.58 (d, 2H, *J* = 7.6 Hz), 5.77 (bs, 1H), 7.15-7.20 (m, 2H), 7.26-7.37 (m, 3H).

### Example 11: further demethylation embodiments of the present invention

The inventors performed the demethylation reaction of the compound of formula (IIa) in different conditions. The results are summarized in Table 1. Said compound of formula (IIa) was weighed in the reaction vessel with a magnetic stirring bar. Sodium thiolate was added all at once followed by the solvent. The mixture was heated to the reaction temperature while stirring. Conversion was monitored by HPLC.

**Table 1**

| | | | | |
|---|---|---|---|---|
| **Thiolate (eq)** | **Solvent** | **T (°C)** | **t** | **Conv.** |
| tBuSNa (8) | DMSO | 80 | 24 h | 100% |
| tBuSNa (5) | DMSO | 80 | 24 h | 100% |
| EtSNa (3) | DMSO | 80 | 60 h | 100% |
| EtSNa (5) | DMF | 100 | 6 h | 100% |

### Example 12: Comparative examples

The inventors of the present invention provide comparative data of the demethylation reaction of the compound of formula (IIa) in different conditions. In particular, the data show the effect of using different ratios of thiolate salt to compound of formula (IIa) and different solvents The results are summarized in Tables 2 and 3.

Said compound of formula (IIa) was weighed in the reaction vessel with a magnetic stirring bar. Sodium thiolate was added all at once followed by the solvent. The mixture was heated to the reaction temperature while stirring. Conversion was monitored by HPLC.

### A) Effect of the molar ratio of thiolate salt

**Table 2**

| **Comparative Example No.** | **Solvent** | **Thiolate** | **Equivalents thiolate** | **Conc. (M)** | **T (°C)** | **t (h)** | **Conv. To product (%)** |
|---|---|---|---|---|---|---|---|
| 11a | DMF | EtSNa | 3.7 | 0.15 | 110 | 24 | Decomp. |
| 11b | DMF | EtSNa | 3.7 | 0.50 | 90 | 4 | 0 |
| 11c | DMF | EtSNa | 3.7 | 0.50 | 100 | 1 | 0 |

From the results obtained, it can be concluded that using molar ratios of thiolate salt: compound of formula (II) lower than the claimed ratio, no demethylation is achieved.

### B) Effect of the solvent

**Table 3**

| **Comparative Example No.** | **Solvent** | **Thiolate** | **Equivalents thiolate** | **Conc. (M)** | **T (°C)** | **t (h)** | **Conv. to product (%)** |
|---|---|---|---|---|---|---|---|
| 11d | ACN | PhSNa | 8 | 0.15 | 80 | 24 | 0 |
| 11e | Dioxane | PhSNa | 8 | 0.15 | 80 | 24 | 0 |
| 11f | Anisole | PhSNa | 8 | 0.15 | 80 | 24 | 0 |
| 11g | ODCB | PhSNa | 8 | 0.15 | 80 | 24 | 0 |
| 11h | ACN | *t*BuSNa | 8 | 0.15 | 80 | 24 | 0 |
| 11i | Dioxane | *t*BuSNa | 8 | 0.15 | 80 | 24 | 0 |
| 11j | Anisole | *t*BuSNa | 8 | 0.15 | 80 | 24 | 0 |
| 11k | ODCB | *t*BuSNa | 8 | 0.15 | 80 | 24 | 0 |

Acetonitrile (ACN), dioxane, and anisole, are known aprotic polar solvents with a polarity index, respectively of 5.8, 4.8, and 3.8. *Ortho*-dichlorobenzene (OCBD) is known as being a non-polar solvent.

As it can be concluded from Table 3, when using solvents other than the polar aprotic solvents referred in claim 1, no demethylation is achieved.

### C) Effect of the starting product

In the prior art, reaction conditions for demethylating quinidine have been disclosed (F. Xu et al., "Assymetric Synthesis of a Potent, Aminopiperidine-Fused Imidazopyridine Dipeptidyl Peptidase IV Inhibitor", 2010, J. Org. Chem. V. 75, p. 1343-1353). Particularly, in said publication the reaction conditions are: quinidine (1.446 mol), EtSNa (5.35 mol), DMF (as solvent), and T110 °C for 6 hours. The molar ratio EtSNa:quinidine was about 3.7.

The use of said reaction conditions to demethylate the compound (IIa) does not work, although both compounds, the quinidine and the compound (IIa) are structurally close.

Thus, the nature of the starting product, and, particularly, the nature of the radical in C9 (in the case of quinidine, the radical is -OH radical, whereas in the compound of formula (II) the radical is -NH₂) seems to have also some influence in the demethylation reaction.

In view of the above, it can be concluded that the specific selection of the starting material, the molar ratio thiolate:compound of formula (II), and the solvent is relevant in order to successfully perform the demethylation of compounds of formula (II) of the present invention.

### REFERENCES CITED IN THE APPLICATION

- JP2010024173A;
- Kacprzak K. et al., "Clickable 9-azido-(9-deoxy)-Cinchona alkaloids: synthesis and conformation", Tetrahedron: Asymmetry, 2010, vol. 21, pp. 2740-2745;
- Franz M. H. et al., "The first and second cinchona rearrangement. Two fundamental transformations of alkaloid chemistry", J. Org. Chem., 2004, vol. 69, pp. 2983-2991;
- Barwick J. V. "Strategies for solvent selection- a literature review", Trends in analytical chemistry, 1997, vol. 6(6), pp. 293-309.
- F. Xu et al., "Assymetric Synthesis of a Potent, Aminopiperidine-Fused Imidazopyridine Dipeptidyl Peptidase IV Inhibitor", 2010, J. Org. Chem. vol. 75, pp. 1343-1353

## Claims

1. A process for preparing a substantially stereochemically pure compound of formula (I), or a salt thereof, 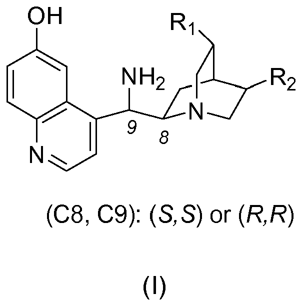 wherein R₁ and R₂ are independently selected from the group consisting of H, -CH₂-CH₃, and -CH=CH₂, in such a way that compound of formula (I) is one of the following compounds: 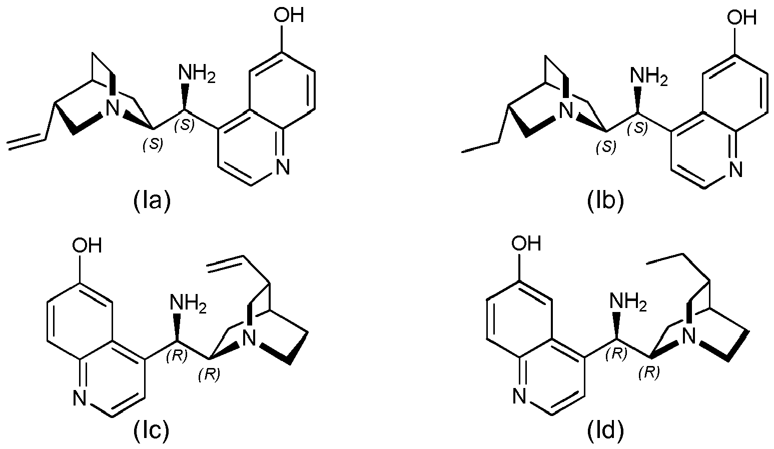 the process comprising the step of demethylating a compound of formula (II), wherein R₁ and R₂ have the same meaning as for compound of formula (I), in the presence of an alkaline or alkaline earth metal (C₁-C₁₀)alkylthiolate salt and a polar aprotic solvent having a polarity index equal to or higher than 6.0, being the molar ratio of thiolate salt:compound of formula (II) comprised of from 2 to 20 equivalents , 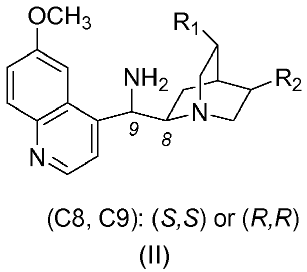 with the proviso that,
when the compound of formula (I) is the compound (Ia), then the compound of formula (II) is the compound (IIa) 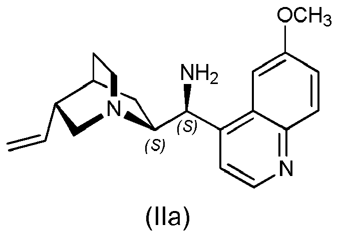 when the compound of formula (I) is the compound (Ib), then the compound of formula (II) is the compound (IIb); 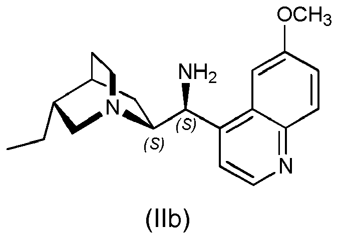 when the compound of formula (I) is the compound (Ic), then the compound of formula (II) is the compound (IIc); and 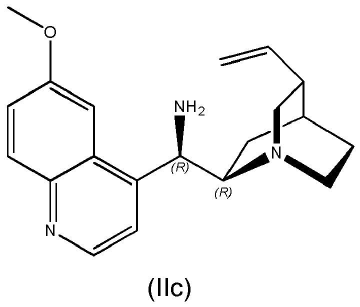 when the compound of formula (I) is the compound (Id), then the compound of formula (II) is the compound (IId)

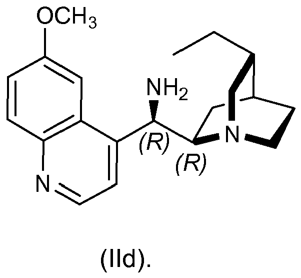

2. The process of claim 1, wherein the polar aprotic solvent is selected from the group consisting of: dimethylformamide, dimethylsulfoxide, methylpyrrolidone, hexamethylphosphoramide, 1,3-Dimethyl-3,4,5,6-tetrahydro-2(1H)-pyrimidinone, N-methylimidazole, 1,1,3,3-tetramethylurea, 1,3-dimethyl-2-imidazolidinone, and mixtures thereof.

3. The process of any one of the claims 1-2, wherein the demethylation takes place at a temperature comprised from 80 to 110°C.

4. The process of any one of the claims 1-3, wherein the molar ratio of the thiolate salt to the compound of formula (II) is comprised of from 4:1 to 12:1.

5. The process of claim 4, wherein the molar ratio of the thiolate salt to the compound of formula (II) is comprised of from 5:1 to 9:1.

6. The process of any one of the claims 1-5, further comprising a previous step wherein the compound of formula (II) is purified by converting it in the corresponding salt by reacting it with an acid and, then, the resulting salt is converted into the corresponding compound of formula (II) by reacting it with a base.

7. The process of claim 6, wherein the preparation of the salt of compound of formula (II) is carried out using an acid, in the presence of a solvent selected from acetonitrile and (C₁-C₁₀)aliphatic alcohol.

8. The process of any one of claims 6-7, wherein the preparation of the salt of the compounds of formula (IIa), (IIc), and (IId) is carried out following the steps:
(i) mixing the compound of formula (IIa), (IIc) or (IId) with a solvent selected from methanol, ethanol, propanol, isopropanol, and *tert-*butanol,
(ii) adding, to the resulting solution of step (i), a solution of an halide acid in a solvent selected from the group consisting of 1,4-dioxane, diethyl ether, methanol, ethanol, and isopropanol, and
(iii) adding to the resulting solution of step (ii) an anti-solvent to provoke the precipitation of the salt.

9. The process of any one of claims 6-7, wherein the preparation of the salt of the compound of formula (IIb) is carried out following the steps:
(i) mixing the compound of formula (IIb) with acetonitrile, and
(ii) adding, to the resulting solution of step (i), a solution of HCl in a solvent selected from the group consisting of 1,4-dioxane, diethyl ether, methanol, ethanol, and isopropanol.

10. The process of any one of claims 5-9, further comprising a previous step wherein a compound of formula (III) 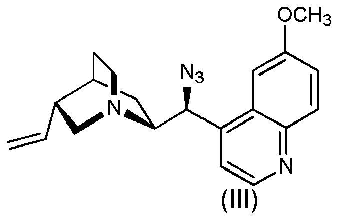 is reduced
(a) either using a metal hydride selected from the group consisting of: lithium aluminium hydride, diisobutylaluminium hydride (DIBAL), Red-Al, borane, borane-diethyletherate and borane-tetrahydrofurane, in the presence of an aprotic polar solvent to yield the compound of formula (IIa), or, alternatively
(b) using a suitable catalyst, in the presence of H₂ and a (C₁-C₁₀)aliphatic alcohol, to yield the compound of formula (IIb).

11. The process of any one of claims 5-9, further comprising a previous step wherein a compound of formula (III') 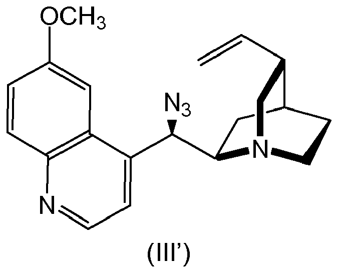 is reduced
(a) either using a metal hydride selected from the group consisting of: lithium aluminium hydride, diisobutylaluminium hydride (DIBAL), Red-Al, and a boron-etherate complex, in the presence of an aprotic polar solvent, to yield the compound of formula (IIc), or, alternatively
(b) using a suitable catalyst, in the presence of H₂, and a (C₁-C₁₀) aliphatic alcohol, to yield the compound of formula (IId).

12. The process of claims 10 or 11, further comprising a previous step wherein a compound of formula (IV) 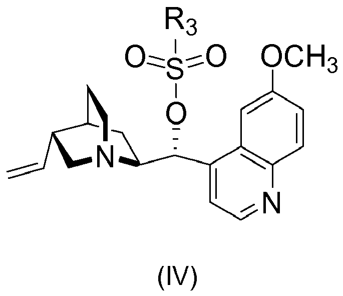 is reacted with an azide source, in the presence of an aprotic polar solvent, to yield the compound of formula (III), or, alternatively
the compound of formula (IV') 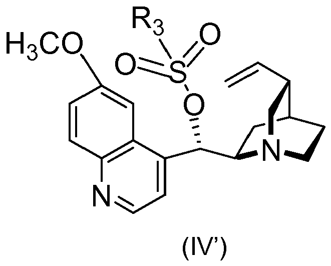 is reacted with an azide salt, in the presence of an aprotic polar solvent, to yield the compound of formula (III'),
wherein, in compounds of formula (IV) and (IV'), R₃ is selected from the group consisting of: (C₁-C₄)alkyl radical, (C₁-C₄)haloalkyl radical, and a phenyl radical optionally substituted by (C₁-C₄)alkyl.

13. The process of any one of claims 10-12, further comprising a previous step wherein a compound of formula (V) 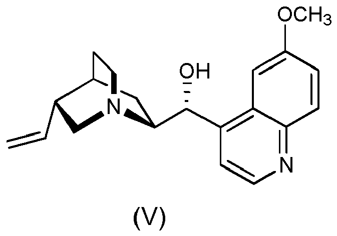 is reacted with a compound of formula (VI)
R₃-SO₂-Z (VI)
wherein
Z is an halide radical or a radical of formula -OSO₂R₄, and
R₃ and R₄ are the same or different and are selected from the group consisting of (C₁-C₄)alkyl radical, (C₁-C₄)haloalkyl radical, and a phenyl radical optionally substituted by (C₁-C₄)alkyl,
in the presence of an aprotic nitrogenated base and a suitable solvent, to yield the compound of formula (IV), or, alternatively
wherein a compound of formula (V') 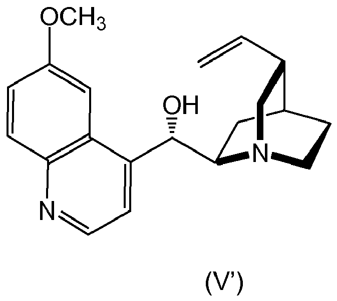 is reacted with a compound of formula (VI) as defined above, in the presence of an aprotic nitrogenated base and a suitable solvent, to yield the compound of formula (IV').

14. A compound of formula (Id):

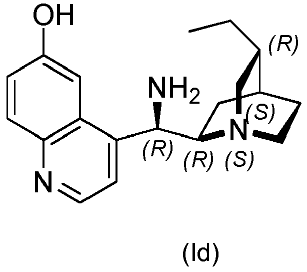

15. Use of a compound of formula (Id) as defined in claim 14 as catalyst.
